# EUROPÄISCHE PATENTSCHRIFT

(11) **EP 0 536 516 B1**
(45) Veröffentlichungstag und Bekanntmachung des Hinweises auf die Patenterteilung: **29.11.1995**
(21) Anmeldenummer: 92114289.9
(22) Anmeldetag: 21.08.1992
(51) Int. Cl.: A61B 17/068

(54) **Gerät zum Setzen von Wundklammern**
Surgical stapler
Agrafeuse chirurgicale

(30) Priorität: 11.10.1991 DE 4133692
(43) Veröffentlichungstag der Anmeldung: 14.04.1993
(73) Patentinhaber: Beiersdorf Aktiengesellschaft, 20245 Hamburg (DE)
(72) Erfinder: Heimerl, Albert, Dr., W-2000 Hamburg 65 (DE); Kartheus, Holger, W-2000 Hamburg 20 (DE); Paske, Dietmar, W-2150 Buxtehude (DE); Plenio, Hans-Ulrich, Dr., W-2100 Hamburg 90 (DE)
(74) Vertreter: Wilcken, Thomas, Dipl.-Ing.

(56) Entgegenhaltungen:
- EP-A- 0 094 752
- EP-A- 0 154 815
- EP-A- 0 203 375
- EP-A- 0 423 589

## Beschreibung

Die Erfindung geht von einem Gerät zum Setzen von Wundklammern aus, die nacheinander von einem Klammerträger auf ein Untergesenk bewegbar und dort mit einem Obergesenk verformbar sind, indem das Obergesenk auf die jeweils zu setzende, auf dem Untergesenk befindliche Klammer absenkbar und weiter verstellbar ist, wobei die Klammer durch Biegen um das Untergesenk verformt und anschließend mit einem Auswerfer vom Untergesenk getrennt wird, der während des Verformungsvorganges in einer ersten Position steht, bei der er sich außer Kontakt mit der jeweils zu implantierenden Klammer befindet, und der nach dem Verformungsvorgang in Richtung auf die implantierte Klammer in eine zweite Position bewegbar ist, um die Klammer vom Untergesenk zu schieben.

Bei bekannten Wundklammergeräten (US-A-3 873 016, US-A-4 109 844, US-A-4 202 480) ist es häufig notwendig, daß der Chirurg das Gerät nach dem Implantieren einer Klammer um eine gewisse Strecke nach vorn oder hinten bewegt, um so die Klammer vom Untergesenk zu lösen. Diese Maßnahme stört jedoch viele Chirurgen, weil die hierzu erforderliche horizontale Bewegung des Gerätes dem sonst beim Klammern hauptsächlich vertikalen Bewegungsablauf entgegengerichtet ist.

Es werden deshalb Geräte bevorzugt, bei deren Anwendung sich der Chirurg nur auf die genaue Plazierung der Klammern konzentrieren muß und nicht weiter durch Bewegungen des Gerätes abgelenkt wird, die zusätzlich zum Abstreifen der jeweils implantierten Klammer vom Untergesenk erforderlich sind. Solche Geräte können nach Setzen einer Klammer einfach vertikal von der Wunde abgehoben werden, nachdem die Klammer selbsttätig aus dem Gerät gestoßen bzw. vom Untergesenk geschoben worden ist.

Aus der EP-B-0 124 556 ist ein Gerät bekannt, dessen Untergesenk mechanisch so verstellbar ist, daß es nach dem Setzen einer Klammer aus dem Bereich zwischen Klammersteg und Wunde zurückgezogen wird, wobei die Klammer vom Untergesenk abgleitet und freigegeben wird. Dieses Gerät hat jedoch eine komplizierte und störanfällige Mechanik. Ein bewegliches Untergesenk hat auch den Nachteil, daß die erforderliche Präzision der Klammerverformung schon bei geringsten und meist nicht vermeidbaren Abweichungen der Iststellung des Untergesenkes von seiner Sollstellung relativ zum Obergesenk nicht mehr gegeben ist.

Eine andere Möglichkeit zum Freisetzen der Klammer vom Untergesenk ist in der EP-A-0 324 166 beschrieben. Bei diesem vorbekannten Gerät drückt eine als Auswerfer dienende Blattfeder ständig auf den Steg der zu implantierenden und implantierten Klammer, so daß die Blattfeder die Klammer vom Untergesenk schieben wird, sobald das Obergesenk aus seiner Anlage mit der Klammer gelangt ist. Diese Lösung hat allerdings den Nachteil, daß durch die Auswerfer-Blattfeder bereits vor und während der Klammerverformung Kräfte auf die zu setzende Klammer einwirken und diese Kräfte dazu führen können, daß die Klammer beispielsweise durch Kippen aus ihrer vorgesehenen Position verstellt wird und dann nicht mehr in exakt ausgerichteter Stellung implantiert werden kann. Deshalb wird es erforderlich sein, die Klammern durch zusätzliche Präzisionsbauteile in mehreren Ebenen zu führen, womit ein entsprechend hoher Aufwand verbunden ist.

Besser wird insofern ein Gerät sein, bei dem der Auswerfer vor und während des Verformungsvorganges in einer ersten Position nicht in Kontakt mit der jeweils zu implantierenden Klammer steht und der nach der Klammerverformung in Richtung auf die implantierte Klammer in eine zweite Position verstellt werden kann, um die Klammer vom Untergesenk zu schieben bzw. abzustreifen.

Bei einem bekannten Gerät dieser Art (US-A-4 523 707) wird der Auswerfer auf einer bogenförmigen Bahn zwischen zwei Positionen verstellt, wobei er beim Einfahren in die erwähnte zweite Position seitlich versetzt wird, um dabei Abstreifwerkzeuge gegen die auf dem Untergesenk befindliche Klammer zur Anlage zu bringen. Die Bewegung des Auswerfers auf einer gekrümmten Bahn und die im Verhältnis hierzu seitliche Versetzbewegung der Abstreifwerkzeuge bedingen allerdings komplizierte Führungen und störanfällige Steuer- und Verstellmechanismen für den Auswerfer. Da dessen Werkzeuge am Klammersteg angreifen, ergeben sich auch ungünstige Hebelverhältnisse im Bereich zwischen den Oberflächen der zu klammernden Wunde und des Untergesenkes, so daß es beim Abstreifvorgang zu einem übermäßigen Kippen und damit zu unerwünschten Bewegungen der Klammer im Gewebe kommen kann.

Durch die Erfindung soll demgegenüber ein Gerät vorgeschlagen werden, dessen Auswerfersystem nach einem einfachen, zuverlässigen und kostengünstigen Prinzip arbeitet und mit dem die Klammer problemlos und ohne Neigung zum Kippen vom Untergesenk geschoben werden kann.

Ausgehend von einem Gerät der einleitend erwähnten Art wird diese Aufgabe so gelöst, daß der Auswerfer auf einer geradlinigen Bahn verstellbar ist und bei Verstellung in seine zweite Position mit einem Kopf unterhalb des Untergesenkes gegen die Schenkel der implantierten Klammer zur Anlage bringbar ist.

Die geradlinige Bewegungsbahn des Auswerfers stellt in konstruktiver Hinsicht geringe Anforderungen an die Führungen und den Verstellmechanismus für den Auswerfer. Wenn dieser unterhalb des Untergesenkes gegen die Klammerschenkel zur Anlage gebracht wird, ergibt sich hieraus ein günstiger Angriffspunkt zwischen Auswerfer und Klammer, da sich dieser Angriffspunkt zwischen der Gewebeoberfläche und dem Bereich befindet, an dem der Klammersteg auf dem Untergesenk liegt. Deshalb wird es beim Abstreifen der Klammer vom Untergesenk vermieden, daß die Klammer kippt und sich hierbei im Gewebe bewegt. Außerdem erfährt der Klammersteg keine Kraftkomponente in Richtung auf das Untergesenk, so daß der Klammersteg im wesentlichen reibungslos vom Untergesenk abgleiten wird.

Der Kopf des Auswerfers hat einen Quersteg, der länger als der Abstand zwischen den Klammerschenkeln ist, um sicher gegen beide Schenkel der Klammer zur Anlage gebracht werden zu können. Wenn der Kopf als ebenes T-Stück ausgebildet ist, muß entsprechend der durch das freie Ende des T-Stückes gebildete Quersteg länger sein als der Abstand zwischen den Klammerschenkeln.

Handbetätigte Geräte zum Setzen von Wundklammern haben meist einen ersten Griff, der zur Betätigung des Gerätes aus einer Ruhelage heraus gegen einen feststehenden zweiten Griff verschwenkt werden kann. Ein solches Gerät kann nach einem weiteren Merkmal der Erfindung eines axialbeweglichen Stößel aufweisen, auf dessen einem Ende ein Nocken des in der Ruhetage befindlichen bzw. in diese Lage zurückkehrenden ersten Griffes einwirkt und dessen anderes Ende mit dem Auswerfer in Kontakt steht.

Im übrigen wird der Klammerträger zweckmäßigerweise von einem feststehenden, aus Blech gestanzten und gebogenen Tragelement abgestützt, dessen distales Ende das Untergesenk bildet und das einen Anschlag aufweist, der auf gleichen Niveau wie der Anschlag am Auswerfer liegt, wobei zwischen beiden Anschlägen eine Feder eingespannt ist, die den Auswerfer ständig in Richtung auf seine erste Position zu drücken sucht, und wobei der am Auswerfer befindliche Anschlag an seiner von der Feder abgekehrten Seite mit dem Stößel zusammenarbeitet.

Schließlich sollte der in einer Wand des Gerätegehäuses geführte Stößel mit einem Teil seiner Länge aus dieser Wand frei nach außen vorstehen und sich dort in der Schwenkebene eines am ersten Griff angeordneten Nockens befinden, so daß der Stößel und damit auch der Auswerfer in Abhängigkeit von der Stellung des ersten Griffes bewegt und gesteuert werden können.

Ein Ausführungsbeispiel der Erfindung ist nachfolgend anhand der Zeichnung näher beschreiben. Es zeigt:
- Figur 1: eine perspektivische Darstellung eines Gerätes nach der Erfindung,
- Figur 2: einen Längsschnitt durch den unteren Bereich des Gerätes nach Figur 1 in größerem Maßstab und bei in zweiter Position befindlichem Auswerfer,
- Figur 3: eine der Figur 2 entsprechende Darstellung, jedoch mit in erster Position befindlichem Auswerfer,
- Figur 4: eine Stirnansicht des Gerätes in Richtung des Pfeiles A in Figur 2 gesehen, wobei einige Geräteteile zum besseren Erkennen anderer Geräteteile weggelassen sind, und
- Figur 5: eine Draufsicht auf den Auswerfer und einige mit diesem zusammenwirkende Teile.

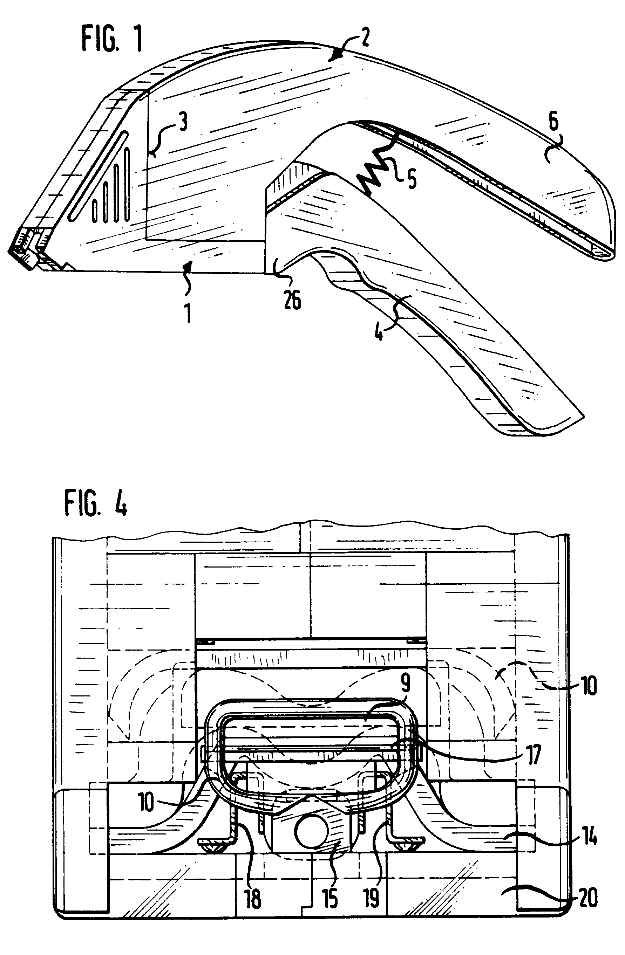

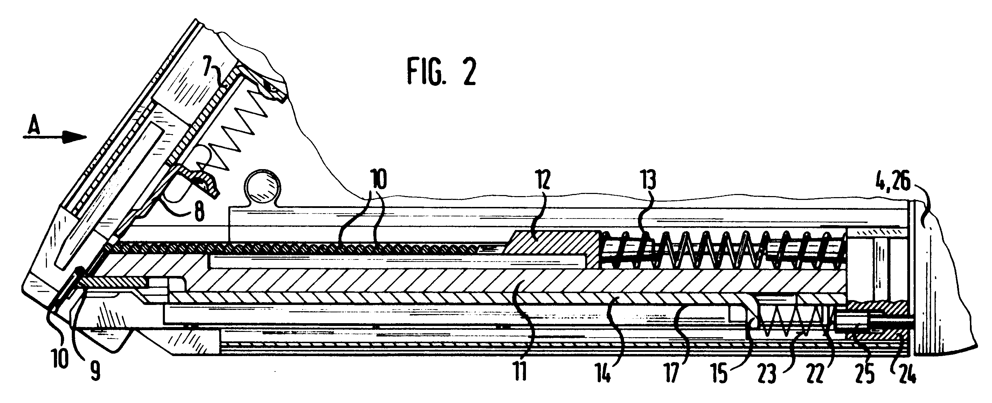

Das Gerät besteht im wesentlichen aus zwei Geräteteilen 1, 2, deren Gehäuse an der Trennebene 3 gegeneinander anliegen. Zum Geräteteil 2 gehört ein erster Griff 4, der gegen eine schematisch dargestellte Druckfeder 5 aus seiner in den Figuren 1 und 2 gezeigten Ruhelage heraus in Richtung auf einen feststehenden, ebenfalls zum Geräteteil 2 gehörenden zweiten Griff 6 verschwenkt werden kann, um zum Implantieren einer Klammer im abnehmbaren Geräteteil 1 befindliche Teile zu betätigen.

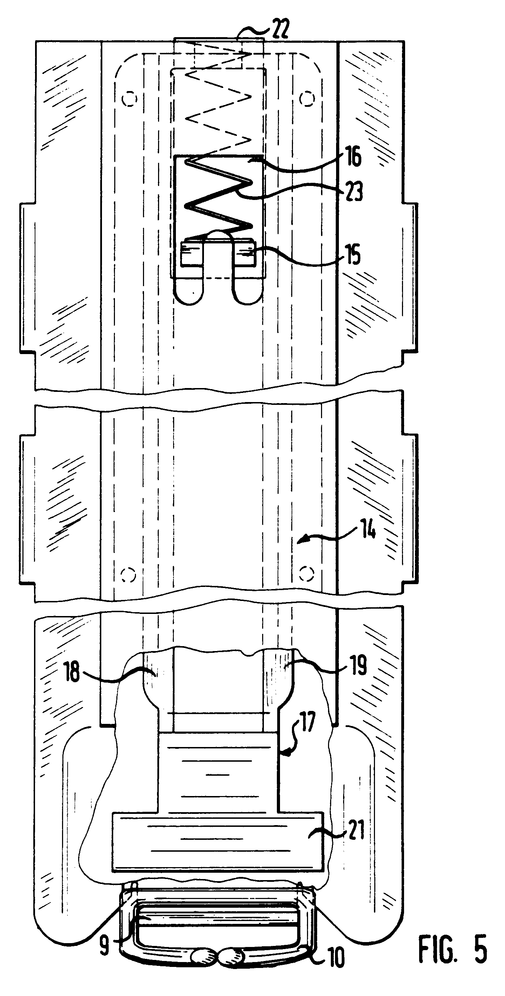

Diese Teile sind u.a. ein Obergesenk 7 und ein Niederhalter 8, die mit ihren Enden durch Betätigung des Griffes 4 aus einer oberen Position (Figur 2) in Richtung auf ein Untergesenk 9 verstellt werden können, um die jeweils auf dem Untergesenk befindliche Klammer 10 mit dem Niederhalter festzulegen und mit dem Obergesenk durch Biegen um das Untergesenk zu verformen, wobei sich die in den Figuren 4 und 5 gezeigte Klammerform ergibt.

Die nachfolgenden Klammern 10 befinden sich auf einem Klammerträger 11 und werden mit einem auf dem Klammerträger geführten Klammertreiber 12 mit einer Druckfeder 13 ständig distalwärts in Richtung auf die Arbeitsebene des Obergesenkes 7 und des Niederhalters 8 gedrückt, so daß die Klammern nacheinander auf das Untergesenk 9 bewegt und dort mit dem Obergesenk 7 verformt werden können.

Der insoweit gegebene Aufbau des Gerätes sowie die Aufgaben und Funktionen der vorerwähnten und sonstigen Teile sind auch in der DE-A-39 34 698 dargestellt und im einzelnen beschrieben worden, so daß sich in diesem Zusammenhang unter Hinweis auf diese Druckschrift weitere Ausführungen erübrigen.

Der Klammerträger 11 wird von einem aus Blech gestanzten und gebogenen Tragelement 14 abgestützt, dessen distales Ende das Untergesenk 9 bildet und an dem ein nach unten abgewinkelter Anschlag 15 vorgesehen ist, der durch eine Ausnehmung 16 eines Auswerfers 17 greift.

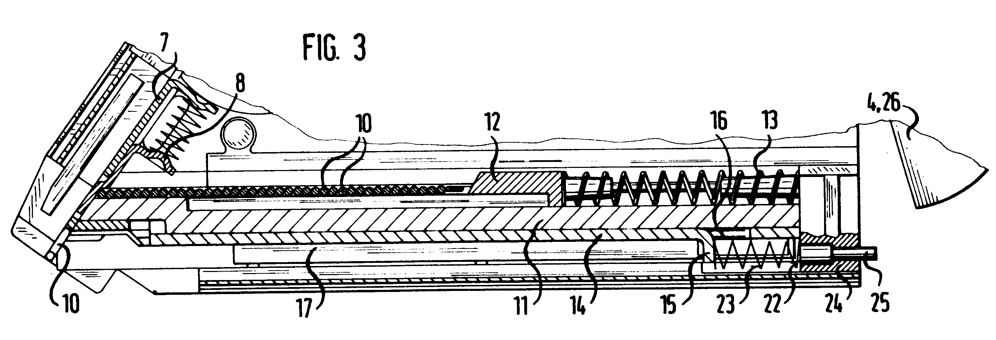

Dieser Auswerfer steht mit Beinen 18, 19 auf einer unteren Gehäusewand 20 des Geräteteiles 1 und ist gleitend längsbeweglich, also gemäß den Figuren 2 und 3 auf einer geradlinigen Bahn nach links und rechts beweglich, unterhalb des Klammerträgers 11 und des Tragelementes 14 geführt, wobei die Führung durch entsprechende Formgebung der nach oben gerichteten Teile des Auswerfers 17 und der unteren, mit diesen Teilen in Anlage befindlichen Kontur des Tragelementes 14 geschaffen wird, wie es anhand der Figur 4 zu erkennen ist.

Der das distale Ende des Auswerfers 17 bildende Kopf 21 ist als T-Stück ausgebildet, dessen Quersteg länger ist als der Abstand zwischen den Schenkeln einer verformten Klammer 10, damit der Auswerferkopf bzw. Quersteg unterhalb des Untergesenkes 9 sicher gegen die Klammerschenkel zur Anlage gelangen und die Klammer vom Untergesenk schieben kann, wie es noch nachfolgend näher beschrieben ist.

Am gegenüberliegenden Ende hat der Auswerfer 17 einen nach oben abgewinkelten Anschlag 22, der auf gleichem Niveau wie der Anschlag 15 am feststehenden Tragelement 14 liegt, so daß zwischen beiden Anschlägen eine Druckfeder 23 eingespannt werden kann, die den Auswerfer ständig in Richtung auf seine erste Position zu drücken sucht, bei der er sich jeweils außer Kontakt mit der auf dem Untergesenk liegenden Klammer befindet (Figur 3).

An der rückseitigen, also von der Feder 23 abgewandten Fläche des Anschlages 22 liegt ein Ende eines in der hinteren Gehäusewand 24 axial beweglich geführten Stößels 25 an, der an seinem anderen Ende mit einem Teil seiner Länge aus der Gehäusewand 24 nach außen vorsteht und sich dort in der Schwenkebene eines am Griff 4 ausgebildeten Nockens 26 befindet.

Bei unbetätigtem Gerät nehmen die Geräteteile die in den Figuren 1 und 2 gezeigten Positionen ein. Wenn man das Gerät durch Verschwenken des Griffes 4 gegen den Griff 6 betätigt, wird anfänglich der Nocken 26 vom Stößel 25 abgehoben, nachdem sich dieser, ausgehend von seiner Stellung gemäß Figur 2, um eine kurze Strecke nach rechts bewegt hat, da die Feder 23 den Auswerfer 17 ebenfalls nach rechts in die zurückgezogene erste Position drückt und dabei der Anschlag 22 den Stößel 25 in seine andere Endposition entsprechend Figur 3 bringt.

Nachdem auf diese Weise der Kopf 21 durch Verstellung des Auswerfers 17 aus der Bewegungsbahn von Obergesenk 7 und Niederhalter 8 sowie aus dem Bereich des Untergesenkes 9 entfernt ist, wird das Obergesenk 7 durch weitergehende Verschwenkung des Hebels 4 und unter Mitnahme der jeweils vordersten Klammer 10 durch den Niederhalter 8 nach unten auf das Untergesenk 9 verstellt, um schließlich die Klammer in bekannter Weise zu verformen und zu implantieren.

Wenn im Anschluß hieran der Griff 4 von Hand freigegeben wird und unter Einfluß der Feder 5 wieder in seine Ruhelage zurückkehrt, werden das Obergesenk 7 und der Niederhalter 8 nach oben in ihre Ausgangsposition zurückgestellt und wird der Nocken 26 gegen Ende dieser Griffbewegung gegen den Stößel 25 zur Anlage Kommen, um diesen zusammen mit dem Auswerfer 17 bei dann größer werdender Spannung der Feder 23 wieder nach links in die zweite mögliche Position gemäß Figur 2 zu bringen. Dabei gelangt der Auswerferkopf 21 im Verlauf dieser Verstellbewegung des Auswerfers 17 gegen die nach unten ragenden Schenkel der implantierten Klammer 10, bis schließlich diese Klammer vollständig vom Untergesenk 9 geschoben ist, wie es die Figur 2 zeigt.

Diese Vorgänge laufen bei entsprechender Auslegung der Feder 5 bis zur vollständigen Trennung der implantierten Klammer vom Gerät selbsttätig ab, so daß der Chirurg zum Freisetzen einer implantierten Klammer einfach nur die das Gerät bedienende Hand zu öffnen braucht. Im übrigen befindet sich der Auswerfer während der Bewegung der zu setzenden Klammer auf das Untergesenk und auch während des Implantierens einer Klammer jeweils in seiner zurückgezogenen ersten Position und demnach außer Kontakt mit der Klammer, so daß der Auswerfer hierbei keine sonst störenden Kräfte auf die Klammer ausüben kann.

Die dargestellten und beschriebenen mechanischen Mittel zum Verstellen des Auswerfers von der einen in die andere Position stellen eine einfache und funktionssichere Lösung dar. Allerdings könnte der Auswerfer auch durch andere bewegliche Teile des Gerätes mit und gegen die Wirkung einer Feder verstellt werden. Im übrigen läßt sich die Erfindung nicht nur bei handbetätigten Geräten verwirklichen, sondern auch bei Geräten mit elektrischen oder anderen Antrieben für die die Klammern verformenden Werkzeuge.

## Patentansprüche

1. Gerät (1, 2) zum Setzen von Wundklammern (10), die nacheinander von einem Klammerträger (11) auf ein Untergesenk (9) bewegbar und dort mit einem Obergesenk (7) verformbar sind, indem das Obergesenk (7) auf die jeweils zu setzende, auf dem Untergesenk (9) befindliche Klammer (10) absenkbar und weiter verstellbar ist, wobei die Klammer (10) durch Biegen um das Untergesenk (9) verformt und anschließend vom Untergesenk (9) mit einem Auswerfer (17) getrennt wird, der während des Verformungsvorganges in einer ersten Position steht, bei der er sich außer Kontakt mit der jeweils zu implantierenden Klammer (10) befindet, und der nach dem Verformungsvorgang in Richtung auf die implantierte Klammer (10) in eine zweite Position bewegbar ist, um die Klammer vom Untergesenk (9) zu schieben, dadurch gekennzeichnet, daß der Auswerfer (17) auf einer geradlinigen Bahn verstellbar ist und bei Verstellung in seine zweite Position mit einem Kopf (21) unterhalb des Untergesenkes (9) gegen die Schenkel der implantierten Klammer (10) zur Anlage bringbar ist.

2. Gerät nach Anspruch 1, dadurch gekennzeichnet, daß der Kopf des (21) Auswerfers (17) einen Quersteg hat, der länger als der Abstand zwischen den Klammerschenkeln ist.

3. Gerät nach einem der vorhergehenden Ansprüche, dadurch gekennzeichnet, daß der Kopf (21) des Auswerfers (17) als ebenes T-Stück ausgebildet ist, dessen Querschnitt länger als der Abstand zwischen den Klammerschenkeln ist.

4. Gerät nach einem der vorhergehenden Ansprüche mit einem ersten Griff (4), der zur Betätigung des Gerätes aus einer Ruhelage heraus gegen einen feststehenden zweiten Griff (6) verschwenkbar ist, gekennzeichnet durch einen axial beweglichen Stößel (25), auf dessen einem Ende ein Nocken (26) des in der Ruhelage befindlichen bzw. in diese Lage zurückkehrenden ersten Griffes (4) einwirkt und dessen anderes Ende mit dem Auswerfer (17) in Kontakt steht.

5. Gerät nach einem der vorhergehenden Ansprüche, dadurch gekennzeichnet, daß der Klammerträger (11) von einem feststehenden, aus Blech gestanzten und gebogenen Tragelement (14) abgestützt ist, dessen distales Ende das Untergesenk (9) bildet und das einen Anschlag (15) aufweist, der auf gleichem Niveau wie ein Anschlag (22) am Auswerfer (17) liegt, daß zwischen beiden Anschlägen (15, 22) eine Feder (23) eingespannt ist, die den Auswerfer (17) ständig in Richtung auf seine erste Position zu drücken sucht, und daß der am Auswerfer (17) befindliche Anschlag (22) an seiner von der Feder (23) abgekehrten Seite mit dem Stößel (25) zusammenarbeitet.

6. Gerät nach einem der Ansprüche 4 und 5, dadurch gekennzeichnet, daß der in einer Wand (24) des Gerätegehäuses geführte Stößel (25) mit einem Teil seiner Länge aus dieser Wand frei nach außen vorsteht und sich dort in der Schwenkebene eines am ersten Griff (4) angeordneten Nockens (26) befindet.

## Claims

1. An instrument (1, 2) for the setting of wound clips (10), which are movable in succession from a clip carrier (11) onto a lower die (9) and are deformable there by an upper die (7), with the upper die (7) being able to be lowered onto the clip (10) which is to be set in each case and is situated on the lower die (9), and with the upper die (7) being able to be further adjusted, wherein the clip (10) is deformed by bending around the lower die (9) and is then separated from the lower die (9) with an ejector (17), which during the deformation process is in a first position in which it is out of contact with the clip (10) to be implanted in each case, and which after the deformation process is movable into a second position in the direction towards the implanted clip (10), in order to push the clip from the lower die (9), characterised in that the ejector (17) is adjustable on a straight path and on adjustment into its second position is able to be brought to lie with a head (21) beneath the lower die (9) against the shanks of the implanted clip.

2. An instrument according to Claim 1, characterised in that the head (21) of the ejector (17) has a cross-piece which is longer than the space between the clip shanks.

3. An instrument according to one of the preceding Claims, characterised in that the head (21) of the ejector (17) is constructed as a flat T-piece, the cross-section of which is longer than the space between the clip shanks.

4. An instrument according to one of the preceding Claims, with a first handle (4) which, to actuate the instrument, is able to be swivelled from a position of rest towards a fixed second handle (6), characterised by an axially movable plunger (25), at one end of which a cam (26) of the first handle (4) acts, which handle (4) is in the position of rest or is returning into this position, and the other end of which plunger (25) is in contact with the ejector (17).

5. An instrument according to one of the preceding Claims, characterised in that the clip carrier (11) is supported by a fixed support element (14) which is punched and bent from sheet metal, the distal end of which forms the lower die (9) and which has a stop (15) which lies at the same level as a stop (22) on the ejector (17), that between the two stops (15, 22) a spring (23) is clamped which continuously seeks to press the ejector (17) towards its first position, and that the stop (22) situated at the ejector (17) cooperates on its side facing away from the spring (23) with the plunger (25).

6. An instrument according to one of Claims 4 and 5, characterised in that the plunger (25), guided in a wall (24) of the instrument housing, projects freely outwards from this wall with a part of its length and is situated there in the swivel plane of a cam (26) arranged on the first handle (4).

## Revendications

1. Appareil (1, 2) pour appliquer des agrafes médicales (10) aptes à se déplacer successivement d'un support d'agrafes (11) sur une articulation inférieure (9) où elles sont aptes à être déformées par le fait que l'articulation supérieure (7) est apte à s'abaisser sur l'agrafe respective (10) à appliquer, se trouvant sur l'articulation inférieure (9), et est apte à effectuer un déplacement ultérieur, dans lequel l'agrafe (10) est déformée par flexion autour de l'articulation inférieure (9) et est ensuite séparée de l'articulation inférieure (9) à l'aide d'un éjecteur (17) qui se trouve dans une première position pendant le processus de déformation, dans laquelle il se trouve a l'écart de l'agrafe (10) respective à implanter et qui, après le processus de déformation, est apte à se déplacer pour prendre une seconde position dans la direction de l'agrafe implantée (10) pour faire glisser l'agrafe à l'écart de l'articulation inférieure (9), caractérisé en ce que l'éjecteur (17) peut se déplacer sur une voie rectiligne et peut être amené, lors du déplacement dans sa seconde position, avec une tête (21) en dessous de l'articulation inférieure (9) pour venir se mettre en contact avec les branches de l'agrafe implantée (10).

2. Appareil selon la revendication 1, caractérisé en ce que la tête (21) de l'éjecteur (17) possède une nervure transversale dont la longueur est supérieure à la distance séparant les branches de l'agrafe.

3. Appareil selon l'une quelconque des revendications précédentes, caractérisé en ce que la tête (21) de l'éjecteur (17) est réalisée en un élément plan en forme de T dont la section transversale est plus longue que la distance séparant les branches de l'agrafe.

4. Appareil selon l'une quelconque des revendications précédentes, comprenant une première poignée (4) qui, à des fins de maniement de l'appareil, peut pivoter en quittant sa position de repos contre une seconde poignée fixe (6), caractérisé par un coulisseau (25) mobile en direction axiale, sur l'extrémité duquel agit une saillie (26) de la première poignée (4) se trouvant dans la position de repos, respectivement retournant dans cette position, et dont l'autre extrémité se trouve en contact avec l'éjecteur (17).

5. Appareil selon l'une quelconque des revendications précédentes, caractérisé en ce que le support d'agrafes (11) est supporté par un élément de support (14) fixe, découpé dans de la tôle et plié, dont l'extrémité distale forme l'articulation inférieure (9) et qui présente une butée (15) qui se trouve au même niveau qu'une butée (22) sur l'éjecteur (17), en ce qu'un ressort (23) est tendu entre les deux butées (15, 22) qui tente de presser en continu l'éjecteur (17) dans la direction de sa première position, et en ce que la butée (22) se trouvant sur l'éjecteur (17) coopère avec le coulisseau (25) sur son côté détourné du ressort (23).

6. Appareil selon l'une quelconque des revendications 4 et 5, caractérisé en ce que le coulisseau (25) guidé dans une paroi (24) du logement de l'appareil fait saillie librement vers l'extérieur avec une partie de sa longueur par rapport à cette paroi et se trouve à cet endroit dans le plan de pivotement d'une saillie (26) disposée sur la première poignée (4).
